Europäisches Patentamt

European Patent Office

Office européen des brevets

(19)

(11) **EP 1 171 473 B1**

(12) **EUROPEAN PATENT SPECIFICATION**

(45) Date of publication and mention
of the grant of the patent:
**06.07.2005 Bulletin 2005/27**

(51) Int Cl.⁷: **C08B 37/06**, C12P 19/04,
A23L 1/0524, A23C 9/137

(21) Application number: **00918319.5**

(22) Date of filing: **23.03.2000**

(86) International application number:
**PCT/US2000/007776**

(87) International publication number:
**WO 2000/058367 (05.10.2000 Gazette 2000/40)**

(54) **PECTIN HAVING REDUCED CALCIUM SENSITIVITY**

PEKTIN MIT VERMINDERTER CALCIUMREAKTIVITÄT

PECTINE PEU SENSIBLE AU CALCIUM

(84) Designated Contracting States:
**AT BE CH CY DE DK ES FI FR GB GR IE IT LI LU
MC NL PT SE**

(30) Priority: **31.03.1999 US 282507**

(43) Date of publication of application:
**16.01.2002 Bulletin 2002/03**

(73) Proprietor: **CP Kelco ApS**
**4623 Lille Skensved (DK)**

(72) Inventors:
• **HANSON, Karin, Meyer**
**DK-4681 Herfolge (DK)**
• **MOELLER, Flemming**
**DK-6000 Kolding (DK)**

(74) Representative: **HOFFMANN - EITLE**
**Patent- und Rechtsanwälte**
**Arabellastrasse 4**
**81925 München (DE)**

(56) References cited:
WO-A-00/27888        WO-A-89/12648
WO-A-97/03574        GB-A- 1 474 990

• **DAAS P J H ET AL: "Investigation of the
non-esterified galacturonic acid distribution in
pectin with endopolygalacturonase"
CARBOHYDRATE RESEARCH,NL,ELSEVIER
SCIENTIFIC PUBLISHING COMPANY.
AMSTERDAM, vol. 318, no. 1-4, 31 May 1999
(1999-05-31), pages 135-145, XP004181054 ISSN:
0008-6215**
• **ROLIN C: "CALCIUM SENSITIVITY OF HIGH
ESTER CITRUS PECTINS" GUMS AND
STABILISERS FOR THE FOOD
INDUSTRY,GB,IRL PRESS, OXFORD, 1994,
pages 413-422, XP000889738**

**Description**

FIELD OF THE INVENTION

The present invention relates to pectin that provides stabilization of proteins without undesirable increase in viscosity. More particularly, the present invention relates to pectin that has been treated by enzymatic or chemical means to produce pectin that provides protein stabilization while having relatively low sensitivity to calcium.

BACKGROUND OF THE INVENTION

Pectin is a purified carbohydrate product obtained by aqueous extraction of appropriate edible plant material, usually citrus fruits or apples. For example, pectin may be commercially extracted from citrus peels or apple pomace under mildly acidic conditions. Typical pectin manufacture comprises three to four primary steps, namely, extraction from the plant material, purification of the extract, isolation of pectin from the purified solution, and optionally, de-esterification.

Chemically, pectin is the methylated ester of polygalacturonic acid. It consists primarily of D-galacturonic acid and galacturonic acid methyl ester units forming linear polysacchande chains, with the D-galacturonic acid units linked together by β-1,4 glycosidic linkages. Some neutral sugars, such as rhamnose and arabinose, are also commonly present. The polygalacturonic acid is partially esterified with methyl groups, and the free acid groups may be partially or fully neutralized with sodium, potassium, or ammonium ions. The ratio of esterified galacturonic acid groups to total galacturonic acid groups, termed the degree of esterification (DE), affects the properties of pectin, especially its solubility and gel forming characteristics.

Pectin is normally classified according to its degree of esterification, and in this context may be divided into two main categories: high (methyl) ester (HM) pectin and low (methyl) ester (LM) pectin. (For purposes of the present discussion, low ester and low methyl ester may be considered interchangeable terms, as may high ester and high methyl ester). The LM pectins are those in which less than 50% of the carboxyl acid units occur as the methyl ester, while the HM pectins are those in which 50% or more of the carboxyl acid units occur as the methyl ester. For regulatory purposes this nomenclature is enforced by FCC definition, under which any pectin of 50% DE or greater is HM pectin, while anything under a DE of 50% is LM pectin.

HM pectins form gels most readily in the presence of high soluble solids and low pH, while LM pectins characteristically gel in the presence of divalent cations, such as calcium and/or magnesium. Although gelling is a primary characteristic of pectin, it may also be used to improve the stability of food and beverage products. Pectin has particular utility in stabilizing beverages which contain proteins, such as milk proteins, especially at low pH. One category of such beverages is acidified milk drinks, which may be produced by fermentation or by direct acidification. Without being bound by theory, it appears that pectin stabilizes proteins, such as casein, by interacting with the ionic charges found at the surface of such proteins. In the absence of stabilization the protein particles in a beverage may interact with each other to agglomerate, resulting in cloudiness and/or precipitation. Pectin is capable of stabilizing proteins by the interaction of the negatively-charged acid regions along the pectin backbone with the ionic charges on the surface of the protein. Because casein is amphoteric, stabilization may also occur via calcium bridging between the negatively-charged acid regions along the pectin backbone and negatively-charged regions on the surface of a casein particle. However, at low pH, such as below about pH 4.4, casein has a predominantly positive charge, such that calcium bridging becomes minimal and stabilization with pectin occurs primarily or exclusively as a result of electrostatic interaction between pectin and casein.

Food and beverage producers may encounter significant variations in the starting materials from which their products are made. In the case of products, including yogurts and beverages, that contain fruit and/or milk, those variations can include significant changes in pH, protein concentration, and calcium concentration, resulting from variations in processing, variations in raw materials, or both. Stabilizing such products with an amount of pectin sufficient to stabilize a typical or standardized product composition may result in product that is of inferior quality or even unusable when the make-up of a particular product composition exceeds the stabilizing power of the amount of pectin used. This risk can be compensated by 'overdosing' with pectin, that is, by adding an amount of pectin in excess of the amount theoretically needed to stabilize a typical or standard batch. The amount of overdosing varies by producer and application, but can be quite large, for example, by 50%, 100%, or more. However, depending on factors such as pH, solids content, and calcium content of a particular product or product batch, the 'extra' pectin may cause a significant increase in viscosity, sometimes referred to as overdosing viscosity. For example, products which include or are based on milk solids, such as acidified milk drinks, may also contain significant concentrations of calcium ions. Here, the food producer or processor may face a dilemma because the pectins that provide better stabilizing power may also cause significant overdosing viscosity.

Various approaches have been tried or suggested to deal with this problem. Akahoshi et al., U.S. Patent 5,690,975, discusses a method for producing calcium enriched fermented milk or fermented milk drinks. The method contemplates adding HM pectin and "calcium individually together with syrup" into a lactic acid-fermented milk drink and homogenizing the resulting mixture; or, subjecting lactic acid-fermented acidified to homogenization, adding syrup and HM pectin thereto, mixing, adding calcium, and mixing again. In either method, a "block-wise type pectin" may be used as the HM pectin. However, from the viewpoint of the food or beverage producer it would generally be preferable to minimize process changes. It would therefore be desirable to obtain a pectin which could be used with foods and beverages having a relatively high soluble solids content and low pH, that would provide stability without undesirable increases in viscosity, even under overdosing conditions.

Details regarding the $\Delta$CS (Calcium Sensitivity) of citrus pectins are disclosed in C. Rolin, Gums and Stabilizers for the Food Industry, GB, IRL Press, Oxford 1994, pages 413-422.

WO 89/12648 concerns a process for improving the gelling properties of high esterified pectin involving the treatment of high esterified pectin with polygalacturonase, which is also known as pectin depolymerase or pectinase. Polygalacturonase acts on pectate and other galacturonans by catalysing the hydrolysis of 1,4-$\alpha$-D-galactosiduronic linkages in a random manner.

WO 00/27888, which represents prior art under Art. 54(3) EPC in the present case, discloses pectins for stabilizing proteins. On page 12, lines 20-23 it is disclosed that the starting mixture of pectins may treated with polygalacturonase or pectate lyase or other chemical or enzymatic techniques which selectively hydrolyse the backbone of the pectin molecule. While the catalytic action of polygalacturonase on pectate and other galacturonans results in a random hydrolysis of 1,4-$\alpha$-D-galactosiduronic linkages, pectate lyase is an enzyme that results in an eliminative cleavage of pectate to give oligosaccharides with4-deoxy-$\alpha$-D-gluc-4-enuronosyl groups at their non-reducing ends. Pectate lyase also acts on other polygalacturonides, while it does not act on pectin.

SUMMARY OF THE INVENTION

The present invention is directed to a process for making a pectin, said process comprising the step of treating a starting pectin having a degree of blockiness of at least 10 %, a degree of esterification of at least 55 %, and a $\Delta$CS (Calcium Sensitivity) of greater than 0 cp (centipoise), with a pectin lyase under conditions sufficient to produce a processed pectin having a $\Delta$CS that is lower than the $\Delta$CS of said starting pectin.

Preferably, the said starting pectin has a degree of blockiness of at least 15 %, more preferably at least 20 %, and preferably of no more than 30 %, more preferably no more than 25 %.

Further, the starting pectin preferably has a degree of esterification of at least 55 %, such as at least 70 % or at least 80 %, and a more preferred range is 55-80 %, even more preferably 60-72 %.

In addition, the starting pectin preferably has a $\Delta$CS of at least 20 cp, such as at least 200 cp, at least 300 cp, or at least 500 cp, and the processed pectin preferably has a $\Delta$CS of no more than 20 cp, more preferably of 0-20 cp.

The starting pectin preferably has a molecular weight of no more than 240,000, more preferably no more than 150,000, and preferably of at least 100,000.

The present invention further provided a pectin obtainable by the above process.

According to a preferred embodiment, this pectin has a degree of blockiness of at least 10 %, a degree of esterification of at least 55 %, and a $\Delta$CS of no more than 100 cp.

The $\Delta$CS of the pectin preferably is no more than 30 cp, such as no more than 20 cp, no more than 10 cp, no more than 5 cp, or about 0 cp, and is preferably, 0-20 cp, more preferably 0-10 cp.

Preferably, the pectin has a degree of blockiness of at least 15 %, more preferably at least 20 %, and preferably of no more than 30 %, more preferably no more than 25 %.

Further, the pectin preferably has a degree of esterification of at least 55 %, such as at least 60 %, 70 % or 80 %, and more preferably ranges between 55-80 %, with 60-72 % being even more preferred.

The pectin obtainable by the present process preferably has a molecular weight of at least 40,000, more preferably at least 50,000, and even more preferably at least 70,000, and is preferably between 50,000 and 200,000, with 70,000-90,000 being particularly preferred.

In another embodiment, the pectin has a degree of blockiness of between 10 and 15, a degree of esterification of between 70 and 75, and a $\Delta$CS of no more than 18 cp, such as no more than 14 cp, no more than 10 cp, no more than 6 cp, no more than 3 cp, no more than 2 cp, or no more than 1 cp.

In an alternative embodiment, the present invention is directed to a process for making pectin process comprising the steps of

(a) treating a pectin having a degree of blockiness of at least 10 %, a degree of esterification of at least 70 %, and a $\Delta$CS of greater than 0 cp with plant pectin methylesterase under conditions sufficient to produce a de-esterified pectin having a degree of blockiness of at least 10 %, a degree of esterification of at least 55 %, and a $\Delta$CS of greater than 0; and

(b) treating said de-esterified pectin with a pectin lyase under conditions sufficient to produce a pectin having a degree of blockiness of at least 10 % and a ∆CS of no more than 20 cp.

Also, the present invention is directed to a pectin obtainable by the above alternative process.

Furthermore, the present invention is directed to a comestible composition comprising a pectin obtainable by the present processes as defined above, and having a degree of blockiness of at least 10 %, a degree of esterification of at least 55 %, and a ∆CS of no more than 100 centipoise.

The comestible composition may further comprise casein, and preferably is a beverage, more preferably an acidified milk drink.

Even further, the present invention is directed to the use of a pectin obtainable by the present processes as defined above for increasing the storage stability of a comestible composition.

The molecular weight of the pectin of the present invention is at least about 40,000, preferably at least about 50,000, and more preferably at least about 70,000, and may be at least about 200,000. A preferred range of molecular weight is between about 50,000 and about 200,000, with a range of between about 70,000 and about 90,000 being particularly preferred.

In another embodiment, the pectin may have a degree of blockiness of between about 10 and about 15, a degree of esterification of between about 70 and about 75, and a ∆CS of no more than about 18. The ∆CS may alternatively be no more than about 14, or no more than about 10, or no more than about 6, or no more than about 3, or no more than about 2, or no more than about 1.

In yet another embodiment, the present invention is directed to a process for making a pectin, including the step of treating a starting pectin that has a degree of blockiness of at least about 10%, a degree of estenfication of at least about 55%, and a ∆CS of greater than about 0 centipoise, with a pectin lyase under conditions sufficient to produce a processed pectin having a ∆CS that is lower than the ∆CS of the starting pectin. The starting pectin may have a degree of blockiness of at least about 20%; alternatively, the starting pectin may have a degree of blockiness of no more than about 30%. Preferably the degree of blockiness of the starting pectin is between about 15% and about 25%.

The degree of esterification of the starting pectin may be at least about 55%, or at least about 70%, or at least about 80%. A preferred range for the degree of blockiness of the starting pectin is between about 55% and about 80%, and a more preferred range is between about 60% and about 72%.

The starting pectin may have a ∆CS of at least about 20, or at least about 200, or at least about 300, or at least about 500.

The processed pectin produced by this process has a ∆CS of no more than about 20 centipoise, preferably no more than about 10 centipoise, even more preferably no more than about 5 centipoise, and most preferably about 0 centipoise. A preferred range of ∆CS for the processed pectin is between about 0 centipoise and about 20 centipoise, with a range of between about 0 centipoise and about 10 centipoise being most preferred.

The starting pectin may have a molecular weight of no more than about 240,000, and preferably of no more than about 150,000, and should have a molecular weight of at least about 100,000.

DETAILED DESCRIPTION OF THE INVENTION

By means of the present invention it is possible to obtain pectins that provide good stabilizing power, without substantial and undesirable increases in viscosity, even when used in relatively high concentrations.

Previously the properties of protein stabilization and minimal viscosity increase generally represented conflicting goals in pectin. Without being bound by theory, this may result from protein stabilization being provided by the presence of negative charges on the pectin, primarily in the form of acid groups, which are also responsible for the divalent cation bridging or cross-linking that produces increases in viscosity up to and including gelling. Therefore, increased amounts of acid groups, which would provide improved stabilization, would also increase the tendency of the pectin to add viscosity to liquids that contain divalent cations, such as calcium. Conversely, selecting pectins having an appropriately low acid content could avoid significant viscosity increase, but would also produce relatively poor stabilizing power. In theory, at least, it may be possible to select pectin which represents a middle ground between these two extremes and provides some stabilizing power without substantial viscosity increase. However, in practice the usefulness of such pectins would be negated by the need for overdosing discussed previously, because under overdosing conditions this compromise in properties breaks down, and undesirable viscosity increases take place.

The present inventors have discovered pectins that provide good stability without, surprisingly, causing undesirable or unacceptable increases in viscosity, even under overdosing conditions The pectins of the present invention may be characterized by various properties, including degree of esterification, or %DE, degree of blockiness, or DB, calcium sensitivity, or ∆CS, molecular weight; protein stabilizing power, or YOG, viscosifying power, or VIS; and by the ratio of viscosifying power to stabilizing power, or VIS/YOG. These parameters, and methods for their measurement, will be further discussed and explained below.

**Degree of Esterification (%DE) :** as previously explained, the degree of esterification of a pectin is a measure of the percentage of acid groups which are present in the pectin molecule as the methyl ester. For HM pectins at least 50% of the carboxyl acid units are present as the methyl ester. As the %DE increases, the calcium sensitivity decreases and, by expectation, so would stabilizing power. The pectins of the present invention are characterized by a %DE of at least about 55, with a range of about 60 or 70 to about 72 being preferred, and the %DE may be as high as about 80. At these higher ranges in particular, stabilizing power would be expected to be poor, with one theoretical basis for this expectation being that there is insufficient negative charge from the relatively few acid groups remaining to effectively stabilize the protein. It should be noted that, when the pectin of the present invention is produced by treating a pectin starting material with enzyme, the %DE of the starting material is essentially unchanged.

Sample Preparation. Weigh 2.0000 g of pectin into a 250 ml glass beaker; add 100 ml of acid alcohol (100 ml 60% isopropyl alcohol plus 5 ml fuming 37% HCl), and stir on a magnetic stirrer for 10 minutes. Filter the resulting solution through a dried, weighed glass filter crucible. Rinse the beaker completely with $6 \times 15$ ml acid alcohol and filter the rinse as well. Wash the filtrate with 60% isopropyl alcohol (IPA) until the filtrate is determined to be chloride-free, as determined by transferring approximately 10 ml of filtrate to a test tube, adding approximately 3 ml of 3 N $HNO_3$, and adding a few (such as 2 or 3) drops of $AgNO_3$. This filtrate may be considered chloride-free if the solution is clear, as opposed to observing precipitation of silver chloride. Typically about 500 ml of IPA wash will be sufficient. Once the filtrate is determined to be chloride-free, wash it with an additional 20 ml of 100% IPA, and dry for 2.5 hours at 105° C. Weigh the crucible after drying.

Measurement : Weigh out two samples of the filtrate, each 0 4000 g, into separate 250 ml glass beakers ; the second sample is a check on the first, and the same procedure is then followed for each sample. Wet the pectin with approximately 2 ml of 100% IPA, and add approximately 100 ml of deionized water while stirring on a magnetic mixer. The sample is now ready for titration. Perform the same procedure on the second sample, recording the second titration amount as $B_1$.

Titration

1. Titrate with 0.1 N NaOH to pH 8. 5, record the titration amount as $V_1$ titer.
2. Add 20.00 ml of 0.5 N NaOH, and allow to stand untouched for 15 minutes covered with foil.
3. Add 20.00 ml of 0.5 N HCl and stir on a magnetic stirrer until the pH is constant.
4. Titrate with 0.1 N NaOH to a pH of 8.5; record the result as $V_2$ titer.

For the blind test:

1. Titrate 100 ml of deionized, carbon dioxide-free water to pH 8.5 with 0.1 N NaOH.
2. Add 20.00 ml of 0.5 N NaOH and allow to stand untouched for exactly 15 minutes covered with foil.
3 Add 20.00 ml 0.5 N HCl and stir on a magnetic stirrer until the pH is constant.
4. Titrate with 0.1 N NaOH to a pH of 8.5; record the result as $B_1$ titer.

The maximum amount allowed for titration in step 4 above is 1 ml of the 0.1 N NaOH. If more than 1 ml of 0.1 N NaOH is required, repeat the procedure, diluting the 0.5 N HCl from step 3 with a small amount of deionized water; similarly, if the sample pH does not fall below 8.5 on addition of the 0.5 N HCl in step 3 above, dilute the 0.5 N NaOH in step 2 with a small amount of deionized water. When dilution is necessary, the maximum allowed dilution is such that the resulting solution is between 0.52 and 0.48 N.

Calculation. The degree of esterification is calculated according to the following formulae:

$$V_t = V_1 + (V_2 - B_1)$$

$$\%DE = \frac{(V_2 - B_1) \times 100}{V_t}$$

**Degree of Blockiness (DB):** the degree of block mess is a measure of the distribution of the acid groups along the pectin molecule. Blockiness refers to the property of acid groups being clustered together in blocks as opposed to being distributed relatively randomly along the polymer. When the acid groups are distributed randomly their individual negative charges are relatively weak, and so would be expected to have little effect in either stabilizing protein or increasing viscosity. However, when the acid groups occur in block-wise fashion, the negative charges associated with the blocks are relatively large, increasing the stabilizing and viscosifying power of the pectin. For purposes of the present description, % DB is expressed as the amount of non-methylated galacturonic acid molecules (mono, di, and trimer) liberated by treatment with endo polygalacturonase (PG, EC 3.2.1.15), as a percentage of the total number of non-estenfied galacturonic acid molecules per gram of pectin. The pectins of the present invention have a %DB that ranges from about 10 to about 30. As with %DE, when the pectin of the present invention is produced by treating a pectin starting material with enzyme, the %DB of the starting material is substantially conserved in the product

<u>Principle and calculations</u>. Polygalacturonase is known to split the pectin polymer by hydrolytic cleavage of the galacturonic chain. It only attacks non-methylated galacturonic acid residues, which means that the release of mono-galacturonic acid, di-galacturonic acid, and tri-galacturonic acid from a pectin polymer depends on (a) the amount of non-methylated galacturonic acid residues present, and (b) the intramolecular distribution of the non-methylated galacturonic acid residues. The larger the amount of non-methylated galacturonic acid residues present in the pectin, the larger the amount of mono-, di-, and trimer that will be released from the pectin polymer by incubation with PG, if the intramolecular distribution of the non-methylated galacturonic acid residues is kept constant. The more clustered or blocky the non-methylated galacturonic acid residues are, the larger the amount of mono-, di-, and trimer that will be released from the pectin polymer by incubation with PG, if the amount of non-methylated galacturonic acid residues is kept constant

For a pectin with a given %DE, the blockiness, as %DB, was determined by quantification of non-esterified mono-, di-, and trimers of galacturonic acid released after treatment with PG. Quantification was performed by high-performance anion-exchange chromatography (HPAEC) as described by Daas, P.J.H., et al , <u>Anal. Biochem.</u>, 257 (2) pp. 195-202 (1988), which is hereby incorporated by reference thereto.

<u>Polygalacturonase preparation</u>. The polygalacturonase preparation used herein, PG-1, was derived from *A. niger* . While the PG-1 used herein was produced by cloning in connection with the EU-funded project "AIR2-CT941345", the enzyme occurs naturally in *A. niger* and may be isolated from *A. niger* using conventional enzyme isolation and purification techniques as previously referenced.

<u>Polygalacturonase activity</u>. The PG activity was assayed as follows :260 :1 (as used herein, the symbol ".1" means microliters) of a polygalacturonic solution (poly-D-galacturonic acid $(C_6H_8O_6)_{n\ ca\ 150}$ USB) was added to 210 : 1 of 50 mM sodium acetate and the temperature of the resulting solution was adjusted to 30°C. Next, 50 :1 of the enzyme to be analyzed was added. The solution was mixed carefully. After 0, 1, 2, 3, 4, and 5 minutes, respectively, a sample of 50 :1 was taken. The reaction was stopped immediately by pipetting the sample into 1 ml of $Na_2CO_3$ (26.5 g/l). After shaking the sample carefully, 0.5 ml of a neucoproin solution (400 mg. neucoproin-hydrochloride CR, $C_{14}H_{13}ClN_2I$ + 200 mg $CuSO_4$ , 5 $H_2O$ in 1 liter ion exchanged water) was added. The sample was incubated at 65°C for 15 mm and left at room temperature for 15 min. The preparation was then applied to a cuvette and absorbency was measured at 460 nm against the sample taken at 0 min. To determine the amount ($\mu$mol) of reducing end groups, a standard curve was prepared from a solution of D-galacturomc acid (8.57 mg/ml). Aliquots of 0, 15, 30, 45, and 60 :1 of the galacturonic acid solution were diluted with 50 mM sodium acetate up to 1 ml. Then, 50 :1 of each sample was treated as described above, starting from "The reaction was stopped... ". A standard curve was made by plotting $\mu$mol galacturonic acid versa absorption measured at 460 nm. The activity was expressed as

$$1U\ (unit) = \mu mol\ reducing\ end\ groups\ produced \times min^{-1}/ml$$

<u>Treatment with PG prior to chromatographic analysis</u>. An amount of 250 mg of the pectin to be analyzed was moistened with 40 $\mu$l ethanol and dissolved in 50 ml of ion exchanged water at 70°C by stirring on a magnetic stirrer. The pectin sample was cooled to 40°C, and the pH was adjusted to 4.2. Next, 1 5 ml of the pectin preparation was added to 3.5 units of PG-1. The sample was incubated at 40°C for 30 min. with constant mixing. The reaction was stopped by addition of 70 $\mu$l $HNO_3$ and heating to 80°C for 10 mm.

<u>Chromatographic analysis</u>. The pectin digests obtained after PG treatment were analyzed as descnbed by Daas, P.J.H. et al., with minor modifications as described below.

A Dionex (Sunnyvale, CA, USA) DX-500 high-performance liquid chromatography (HPLC) system, equipped with a GP40 quaternary gradient pump, an AS3500 autosampler, and an eluent degas (He) module was used. Detection was accomplished with a sodium hydroxide post-column delivery system and a Dionex ED40 electrochemical detector with an amperometric cell. The detector was working in the pulsed amperometric detection (PAD) mode and was equipped with a gold working electrode and an AG/AgCl reference electrode. The postcolumn delivery system consisted of a DQP-1 pump, a pulse damper system, and a guard column (GM-3). The outlet was connected to the chromatographic column outlet with a T-junction and mixed in a 1500 $\mu$l mixing coil. After mixing the eluate with sodium hydroxide (2.5 M), the sample entered the PAD detector. Chromatograms were recorded with Dionex Peak Net software version 4.11. Oligomers were separated on a Dionex CarboPac PA 1 column with a CarboPac PA-1 guardcolumn. Elution was performed with a linear gradient from 0.05-0.7 M sodium acetate (pH 5.0) for 65 min at 0.5 ml/min, followed by a 10 min linear gradient 1 M sodium acetate and a wash step of 0.5 ml. After an equilibration step of 15 min. with 0.05 M sodium acetate (pH 5.0), 80 μl samples were injected. The postcolumn pump was adjusted such that the final flow rate to the PAD cell was 1.0 ml/min.

The acetate buffer was prepared by diluting acetic acid in water, titrating with a 50% solution of sodium hydroxide to pH 5.0, and adjusting the volume with water, to provide a final concentration of 1.0 M acetate. The gradient was accomplished by mixing the acetate buffer with Millipore system water.

The triple-pulse sequence used for amperometric detection included the following potentials and durations: $E_1 = 0.05$ V ($t_1 = 0.4$ s), $E_2 = 0.75$ V ($t_2 = 0.2$ s) and $E_3 = -0.15$ V ($t_3 = 0.4$ s). Integration was for 0.2 seconds after a delay of 0.2 seconds.

To quantify the amount of non-esterified mono-, di-, and trigalacturonic acid amounts detected, the PAD-response factors of these components had to be determined (see Hotchkiss A.T. et al., Anal. Biochem., 184, 200-206 (1990)). First, the peak areas of various amounts of di- and trimer (0.05-2.5 μmoles) were obtained and compared with those of identical amounts of monogalacturonic acid. The relative response factors were calculated. During each series, the PAD-response area of a standard amount of mono-galacturonic acid (0.051μm) was determined to enable accurate calculation of the mono-, di- and trigalacturonic acid concentrations in that senes. From the amount of mono-, di-, and trigalacturonic acid, the degree of blockiness was calculated using the formula for %DB set forth above.

**Molecular Weight (MW) :**The (weight average) molecular weight of pectin can fall within a wide range depending on the source and the method used for extraction. For purposes of the present invention, pectin used as a starting material will generally have a molecular weight of less than about 240,000, and preferably in the approximate range of from about 100,000 to about 150,000. (Unless expressly stated otherwise, the molecular weights provided herein are in Daltons). After enzyme treatment the molecular weight of the processed pectin will of course be lower than that of the selected starting pectin, and in general terms will be in the range of from about 40,000 to about 200,000 Daltons. It appears that the efficacy of the processed pectin is substantially reduced below a molecular weight of about 40-50,000, and is somewhat reduced, but still useful, in the range of from about 40-50,000 to about 70,000. A preferred range of molecular weight for the pectin of the present invention is from about 70,000 to about 90,000.

Pectin Sample Preparation. The molecular weight of pectin is estimated by measuring the relative viscosity of a 0.1% pectin solution using sodium hexametaphosphate. First, a sample is prepared as described in the section regarding the determination of %DE. A sodium hexametaphosphate solution is prepared by dissolving 20.0 g of sodium hexametaphosphate in 1800 ml of ion-exchanged, deaerated (boiled) water, adjusting the pH to $4.50 \pm 0.05$ with 1 M HCl, and diluting to 2000 ml with ion exchanged, deaerated (boiled) water). Immediately before measuring, the necessary quantity of hexametaphosphate solution is filtered through a glass filter # 3. Then, approximately 90 g of sodium hexametaphosphate solution is weighed into a tared beaker to which a magnet is added.

Next, 0.1000 g of the acid-washed pectin is added to the beaker gradually while stirring. The solution is heated, with stirring, to 70° C, and stirring is continued until the pectin is completely dissolved. The solution is then cooled to 25° C, brought up to a weight of 100.0 g with the sodium hexametaphosphate solution, and filtered through a glass filter # 3.

Molecular Weight Measurement. For each molecular weight determination, the outlet time is measured for the pectin/hexametaphosphate solution on two different viscosimeters using the below procedure. The molecular weight is calculated separately for each viscosimeter using the measured outlet time for hexametaphosphate solution on the viscosimeter in question. If the difference between the two calculated molecular weights is less than 3500, the mean value is calculated and rounded to the nearest multiple of 1000. If the difference between the two calculated molecular weights is 3500 or more, the viscosimeters should be cleaned and a new measuring of outlet time for hexametaphosphate solution should be performed.

To measure outlet time, the viscosimeter is rinsed twice with the sample. Then, 5.00 ml of the sample is poured in the viscosimeter and placed in a thermostat-equipped water bath at 250°C $\pm$ 0.3 °C at least 15 minutes prior to measuring. The outlet time is measured on two outlets. If the difference between the times is more than 0.2 seconds (when measuring hexametaphosphate solution) or more than 0.4 seconds (when measuring samples), the measurements are repeated until the difference is less than the appropriate value. The outlet time used for the molecular weight determination is the mean value of the above-mentioned identical or substantially identical measuring results.

The relative viscosity is calculated as follows:

$$n_r = \frac{\left(t_o - \dfrac{K}{t_o}\right)}{\left(t_h - \dfrac{K}{t_h}\right)}$$

where $t_o$ and $t_h$ are outlet times for pectin solution and hexametaphosphate solution, respectively.

The parameter K can with sufficient accuracy be fixed at 107 $s^2$ using Witeg-Ostwald-viscosimeter. Otherwise, K can be calculated as follows:

$$K = \frac{Q \times t^2_v}{Q + (0.226 \times L \times t_v)}$$

where Q = volume of viscosimeter bulb in $cm^3$, L = length of capillary tube in cm, and $t_v$ = outlet time for water in seconds.

The molecular weight of pectin is calculated as follows:

$$M = \frac{\left(n_r^{1/P} - 1\right)}{k \times C} \times P$$

where P is fixed at 6 and k is fixed at $4\,7 \times 10^{-5}$ mol$\cdot$ g$^{-1}$, C is the weight percentage of pectin in the sample system. With numerical values inserted, this becomes:

$$M = 1.277\cdot 10^6 \, (n_r^{1/6} - 1) \text{ g/mol.}$$

**Calcium Sensitivity ($\Delta$CS):** the calcium sensitivity of the processed pectin is substantially reduced by means of the present invention, which is particularly surprising given that the DB remains essentially constant. As further explained below, the calcium sensitivity represents the difference in viscosity between a reference solution containing pectin without calcium and a solution containing pectin with calcium, and for this reason may be represented as $\Delta$CS. The pectin of the present invention will have a $\Delta$CS of less than about 100, preferably less than about 20, such as in the range of from about 5 to about 10, and is preferably about 0. The $\Delta$CS of the starting material can be any value over about 20, and can be very high, such as greater than about 300, or even greater than about 500.

The calcium sensitivity of the pectin is measured in a solution with 0.5% pectin, preferably pure pectin. The calcium salt and pectin are mixed at low pH, to avoid any strong setting of the calcium ions and pectin. The reaction between the pectin and the calcium ions is then started by the addition of sodium acetate/acetic acid buffer. The viscosity of the calcium-containing pectin solution is compared with the viscosity of the same solution without calcium, and the difference in viscosity is the calcium sensitivity, or $\Delta$CS, measured in centipoise.

More specifically, a 400 g solution is made with 2.4 g pectin (0.6% solution). If pure pectin is not used, the solution should be corrected by using an amount of pectin calculated according to the formula

$$\frac{0.6 \times 400}{A} \, ,$$

where A is the pectin percentage of the sample.

The amount of pectin to be used (2.4 g if pure, otherwise calculated as above) is weighed out to a precision of three decimal places. The pectin is dispersed into 250 ml of boiling deionized water in a high-shear mixer (Waring Blender Model 34BL99, Waring Product Division Dynamic Corporation of America, New Hartford, Connecticut) at high speed for approximately 4 minutes. (All deionized water used herein is ion exchanged water with a conductivity below 1.0 $\mu$S/cm.) The sheared pectin solution is poured into a tared beaker, and a magnet is added for mixing on a JK IKA-Combimag REO magnetic stirrer (Janke & Kunkel (IKA), Staufen, Germany). The high-shear mixer is rinsed with an additional 100 ml of deionized water, which is added to the pectin solution in the beaker. The solution is cooled to 25 °C $\pm$ 1°, and then the pH is adjusted to 1.5 using 1 M hydrochloric acid while mixing. Additional DI water is added to increase the solution weight to 400 g, and 145 g $\pm$ 1g of pectin solution is weighed into each of two 50 x 100 mm viscosity glasses (Holm & Halby). A magnet is added to each viscosity glass. One of the viscosity glasses then receives 5 ml of deionized water while the other viscosity glass receives 5 ml of 250 mM Ca++ solution (36.7550 g/l of $CaCl_2$, $2H_2O$), both while stirring on a IKA MAG EOA 9 magnetic plate stirrer at Step 1. Next, 25 ml of 1 M acetate buffer (68.04 g/l of 500 mM $CH_3COONa$, $3H_2O$, and 28.6 ml/l of 500 mM $CH_3COOH$, 100%) is added to each viscosity glass while stirring vigorously on an IKA MAG RET magnetic stirrer. Stirring is continued at step 1 for 2 minutes, and the magnets are then removed and the solutions are covered with a Parafilm® film and allowed to sit at 25 °C overnight. Viscosity is then measured with a Brookfield LVT viscosimeter at 60 RPM, still at 25 °C in the thermostatically controlled water bath. The difference between the viscosities of the two solutions, in centipoise, is the calcium sensitivity, or $\Delta$CS, of the subject pectin.

The pectins of the present invention may be produced by treating a starting pectin, as further described herein, with pectin lyase under conditions sufficient to obtain a pectin having the desired properties. The process for making the pectins of the present invention does not significantly affect the degree of blockiness or the degree of esterification of the starting materials. Because the DB relates to how concentrated the acid groups are along the pectin backbone, and concentration of acid groups is generally related to calcium sensitivity, it is quite surprising that calcium sensitivity can be decreased without a change in DB.

Neither the particular pectin lyase used for treatment, nor the source of the pectin used as starting material, appear to be critical, and may be selected or adjusted as a routine matter of process optimization. Pectin lyases are well known, and while other sources are available, they are commonly obtained from Aspergillus niger, in which several variants occur; see, for example, Van Houdenhoven, F.E.A., "Studies on Pectin Lyase", Ph.D. Thesis, Wageningen Agricultural University, The Netherlands, 17 (1975); and, Kusters-Van Someren, M.A., "Characterization of an Aspergillus niger Pectin Lyase Gene Family", Ph.D. Thesis, State University Utrecht, The Netherlands (1991), the disclosures of which are hereby incorporated by reference. The presence of contaminants, and especially biologically active contaminants, in the lyase preparation will detract from the ability to practice the process and obtain the pectins of the present invention, and therefore only lyase preparations that are obtained in substantially pure form or are punfied to substantial homogeneity should be used.

As previously indicated, pectin is present in, and may be extracted from, a wide range of plant sources. These include, without limitation, relatively common commercial sources such as lemon, lime, orange, grape, apple, and sugar beet.

Examples

**Enzymes :** four different enzyme preparations containing pectin lyase (PL, E.C. 4.2.2.10) were used. Preparation A was pectin lyase A (PL A) derived from *Aspergillus niger*, and Preparation B was pectin lyase D (PL D) also derived from *A. niger* . While the samples of PL A and PL D used in the following examples were produced by cloning in connection with the EU-funded project "AIR2-CT941345", these enzymes occur naturally in *A. niger* and may be isolated from *A. niger* using conventional enzyme isolation and purification techniques, such as those described by Van Houdenhoven. The enzyme should be purified to substantial homogeneity in order to maximize activity, and to avoid contamination and/or undesirable side reactions which may result from impure preparations.

Preparation C was a commercial fungal enzyme preparation available as "Pectinase 444 L" from Biocatalysts, United Kingdom, while Preparation D was a commercial enzyme preparation available as "Rohapect PTE", prepared from genetically modified organisms (Aspergillus species), by Röhm Germany.

**Enzyme Activity:** enzyme preparations A to D were all tested for PL activity. PL catalyses the non-hydrolytic breakdown of pectin by a trans-eliminative split of the polymer backbone at C-4, and simultaneously eliminates the H from C-5, creating a double bond between C-4 and C-5, which causes an increase in absorbency at 235 nm. Pectin lyase activity was accordingly assayed spectrophotometrically at 30° C by measuring the increase in optical density at 235 nm. The assay mixture contained 1.4 ml McIlvaine buffer (36.85 mM citric acid, 126.3 mM $NaH_2PO_4$, pH adjusted to 5.5 with 0.5 N NaOH), 0.4 ml 0.0625 % pectin (B-rapid set having a DE of 72%, Copenhagen Pectin A/S, Denmark), and 0.2 ml of enzyme. The activity was calculated by using the molar extinction coefficient $\Sigma_{235}$ 5500. One unit of activity (1U) was determined as the amount of enzyme which creates 1 $\mu$mol of unsaturated product/minute.

When the procedures described herein refer to an "appropriate amount" of pectin lyase (or words to that effect), this is an amount calculated based on the target molecular weight of the lyase-treated pectin and the specific activity of the lyase being used. The amount of enzyme to be used is given by the equation $e^{(y-a/b)}=x$, where y is the reduction in molecular weight (therefore $\Delta$MW), and x is the amount of enzyme (in units) per 500 grams of pectin. In using this equation, the process time for enzyme treatment is taken as one hour, a and b are enzyme-specific constants determined after running several trials with the enzyme in question.

**Enzymatic Treatment:** three different starting materials were subjected to enzymatic treatment of pectin with preparations containing PL ; juice derived from dried peel by HM extraction, juice treated with plant methyl esterase (PME), and dried pectin powder.

Juice derived from dried peel by HM extraction. Dried lemon peel in the amount of 600 kg was added to 1700 liters of ion exchanged water. The preparation was heated to 70°C and stirred for 2 hours ; then 98 liters of 62 % $HNO_3$ was added, and extraction was carried out for 1 hour. Next, 100 kg of paper pulp was added to the extracted material in order to facilitate filtration. The preparation was vacuum and filtered with diatomaceous earth ("blank filtered"). The filtrated juice was ion exchanged on a strong cation exchanger (IRC 120, Bayer Corporation); evaporated to about 50% of the original volume, resulting in a pectin concentration of from about 3 to about 5% on a weight/weight dry weight basis ; and cooled to room temperature. The pH was adjusted to 5.5 by addition of $NH_3$ (2% solution, diluted with DI water from 25% $NH_3$), and an appropriate amount of PL was added.

The enzyme reaction was carried out until the desired molecular weight was achieved. In order to stop the reaction, the pH was lowered to 2.5 by addition of diluted (10% v/v) $HNO_3$, and the temperature was raised from room temperature to 80°C for 10 minutes using a heating jacket. The pectin was precipitated at a ratio of 1.3 in 80% 2-propanol. The precipitated pectin was pressed and washed in 60% 2-propanol. After pressing, the pectin was dried at 70°C for 16-20 hours. The dried sample was ground and sieved through a 0.25 mm-test sieve.

Juice treated with PME. Dried lemon peel in the amount of 1,300 kg was added to 25,500 liters of ion exchanged water and 126 liters of 62% $HNO_3$. Extraction was carried out for 7 hours. Paper pulp (100 kg) was added to the extracted material in order to facilitate filtration. The preparation was vacuum filtrated, followed by a blank filtration. The filtrated juice was ion exchanged on the strong cation exchanger (IRC 120); evaporated to about 50% of the original volume, resulting in a pectin concentration of from about 3 to about 5% on a weight/weight dry weight basis; cooled to 45°C, and the pH was adjusted to 5.5 by addition of 2% $NH_3$.

An appropriate amount of plant PME was added and the reaction was carried out until the desired degree of estenfication was achieved. The desired degree of esterification is determined by the relationship y=1.6x, where y is the change in %DE (therefore, Δ%DE), and x is grams of $NH_3$/kilogram of pectin (g $NH_3$/kg pectin). During the reaction the pH was kept constant by automatic titration with 2% $NH_3$. The PME was inactivated by addition of $HNO_3$ to a pH of 2.5 and heating to 80°C for 10 min. After cooling to room temperature, the pH was adjusted to 5.5 with a 2% v/v solution of $NH_3$, and an appropriate amount of PL was added. The enzymatic reaction was allowed to proceed until the desired molecular weight was achieved. In order to stop the reaction, the pH was lowered to 2.5 by addition of diluted $HNO_3$ and the temperature was raised to 80°C for 10 min. The pectin was precipitated in 80% 2-propanol in a 1:3 ratio of pectin to propanol. The precipitated pectin was then pressed and washed in 60% 2-propanol. After pressing, the pectin was dried at 70°C for 16-20 hours. The dried sample was ground and sieved through a 0.25 mm-test sieve.

Dried pectin powder. Pectin in the amount of 500 g was dissolved in 10 liters of ion-exchanged water at 70°C with vigorous stirring. The pectin solution was cooled to room temperature and the pH was adjusted to 5.50 by the addition of 2% $NH_3$. An appropriate amount of PL was added, and the enzyme reaction was allowed to proceed until the desired molecular weight was achieved. In order to stop the reaction, the pH was lowered to 2.5 by the addition of diluted $HNO_3$, and the temperature was raised to 80°C for 10 min. Pectin was precipitated (1:3) in 80% 2-propanol. The precipitated pectin was pressed and washed in 60% 2-propanol. After pressing, the pectin was dried at 70°C for 16-20 hours. The dried sample was ground and sieved through a 0.25 mm test sieve.

The results are shown below:

Example 1 - PL D

| Material | DB | DE | ΔCS | MW | YOG C | VIS C | YOG/VIS |
|---|---|---|---|---|---|---|---|
| Starting Pectin | 13 | 72 | 298 | 144 | 169 | 148 | 88 |
| PL D-1A | 13 | 72 | 1 | 81 | 101 | 66 | 65 |
| PL D-2A | 13 | 72 | 1 | 67 | 94 | 0 | 0 |
| PL D-3A | 13 | 72 | 2 | 42 | 48 | 0 | 0 |
| PL D-4A | 13 | 72 | 1 | 34 | SEP | SEP | SEP |
| PL D-5A | 13 | 72 | 2 | 25 | SEP | SEP | SEP |

Example 2 - PL A

| Material | DB | DE | ΔCS | MW | YOG C | VIS C | YOG/VIS |
|---|---|---|---|---|---|---|---|
| Starting Pectin | 13 | 72 | 298 | 144 | 169 | 148 | 88 |
| PL A-1A | 13 | 71 | 90 | 108. 5 | 142 | 70 | 49 |
| PL A-2A | 13 | 71 | 30 | 95 | 88 | 63 | 72 |
| PL A-3A | 13 | 72 | 6 | 94.5 | 84 | 42 | 50 |
| PL A-4A | 13 | 72 | 1 | 71.5 | 37 | 0 | 0 |
| PL A-5A | 13 | 72 | 0 | 41.5 | SEP | SEP | SEP |

Example 3 - 444L

| Material | DB | DE | ΔCS | MW | YOG C | VIS C | YOG/VIS |
|---|---|---|---|---|---|---|---|
| Starting Pectin | 13 | 72 | 298 | 144 | 169 | 148 | 88 |
| 444L-1A | 13 | 71 | 14 | 106 | 126 | 104 | 82 |
| 444L-2A | 13 | 72 | 3 | 96 | 112 | 94 | 83 |
| 444L-3A | 13 | 71 | 1 | 83 | 96 | 52 | 54 |
| 444L-4A | 13 | 72 | 2 | 70 | 75 | 39 | 52 |
| 444L-5A | 13 | 72 | 3 | 70 | 72 | 35 | 49P |

Example 4 - PTE

| Material | DB | DE | ΔCS | MW | YOG C | VIS C | YOG/VIS |
|---|---|---|---|---|---|---|---|
| Starting Pectin | 13 | 72 | 298 | 144 | 169 | 148 | 88 |
| PTE-1A | 13 | 71 | 18 | 102 | 117 | 91 | 78 |
| PTE-2A | 13 | 72 | 18 | 99 | 109 | 78 | 72 |
| PTE-3A | 13 | 72 | 10 | 71 | 75 | 52 | 69 |
| PTE-4A | 13 | 70 | 3 | 66 | 49 | 55 | 112 |
| PTE-5A | 13 | 72 | 1 | 48 | SEP | SEP | SEP |
| PTE-6A | 13 | 71 | 1 | 37 | SEP | SEP | SEP |

While these results were obtained using enzymatic treatment, comparable results may be obtained using chemical treatment, provided that the treatment does not alter the %DE and the %DB of the starting pectin. In particular, any chemical treatment which causes cleavage of the 1,4 glycosidic linkage via beta elimination may be used to produce pectin having properties comparable to the pectin produced by enzymatic treatment characterized above. For example, treatment of a pectin solution with an alkaline reagent, such as NaOH, while elevating the pH of the solution to above 7, should produce similar results.

The present invention has of necessity been discussed herein by reference to certain specific methods and materials. The enumeration of these methods and materials was merely illustrative, and in no way constitutes any limitation on the scope of the present invention. It is to be expected that those skilled in the art may discern and practice variations of or alternatives to the specific teachings provided herein, without departing from the scope of the present invention.

**Claims**

1. Process for making a pectin, said process comprising the step of treating a starting pectin having a degree of blockiness of at least 10 %, a degree of esterification of at least 55 %, and a ∆CS (Calcium Sensitivity) of greater than 0 centipoise, with a pectin lyase under conditions sufficient to produce a processed pectin having a ∆CS that is lower than the ∆CS of said starting pectin.

2. The process of claim 1, wherein said starting pectin has a degree of blockiness of at least 20 %.

3. The process of claim 1 or 2, wherein said starting pectin has a degree of blockiness of no more than 30 %.

4. The process of any of the claims 1-3, wherein said starting pectin has a degree of esterification of at least 55 %.

5. The process of claim 4, wherein said starting pectin has a degree of esterification of between 55 % and 80 %.

6. The process of claims 1-5, wherein the starting pectin has a ∆CS of at least 20.

7. The process of any of claims 1-6, wherein said processed pectin has a ∆CS of no more than 20 centipoise.

8. The process of claim 7, wherein said processed pectin has a ∆CS of between 0 centipoise and 20 centipoise.

9. The process of any of claims 1-8, wherein said starting pectin has a molecular weight of no more than 240,000.

10. Pectin obtainable by the process of any of claims 1-9.

11. Pectin of claim 10, having a degree of blockiness of at least 10 %, a degree of esterification of at least 55 %, and a ∆CS of no more than 100 centipoise.

12. The pectin of claim 11, having a ∆CS of no more than 30 centipoise.

13. The pectin of claim 12, having a ∆CS of between 0 centipoise and 20 centipoise.

14. The pectin of any of claims 10-13, having a degree of blockiness of at least 20 %.

15. The pectin of claim 14, having a degree of blockiness of no more than 30 %.

16. The pectin of any of claims 10-15, having a degree of esterification of at least 60 %.

17. The pectin of claim 16, having a degree of esterification of up to 80%.

18. The pectin of claim 17, having a degree of esterification of between 60 % and 72 %.

19. The pectin of any of claims 10-18, having a molecular weight of at least 40,000.

20. The pectin of claim 19, having a molecular weight of between 50,000 and 200,000.

21. The pectin of any of claims 10-20, having a degree of blockiness of between 10 and 15, a degree of esterification of between 70 and 75, and a ∆CS of no more than 18.

22. The pectin of claim 21, having a ∆CS of no more than 14.

23. A process for making pectin, said process comprising the steps of

(a) treating a pectin having a degree of blockiness of at least 10 %, a degree of esterification of at least 70 %, and a ∆CS of greater than 0 centipoise with plant pectin methylesterase under conditions sufficient to produce a de-esterified pectin having a degree of blockiness of at least 10 %, a degree of esterification of at least 55 %, and a ∆CS of greater than 0; and

(b) treating said de-esterified pectin with a pectin lyase under conditions sufficient to produce a pectin having a degree of blockiness of at least 10 % and a ΔCS of no more than 20 centipoise.

24. Pectin obtainable by the process of claim 23.

25. A comestible composition comprising a pectin according to any of claims 10-22 and 24 having a degree of blockiness of at least 10 %, a degree of esterification of at least 55 %, and a ΔCS of no more than 100 centipoise.

26. The comestible composition of claim 25, further comprising casein.

27. The comestible composition of claim 26, wherein said comestible composition is a beverage.

28. The comestible composition of claim 27, wherein said beverage is an acidified milk drink.

29. The comestible composition of any of claims 25-28, wherein said pectin has a ΔCS of no more than 20 centipoise.

30. The comestible composition of any of claims 25-29, wherein said pectin has a degree of blockiness of between 15 % and 25 %.

31. The comestible composition of any of claims 25-30, wherein said pectin has a degree of esterification of between 60 % and 72 %.

32. The comestible composition of any of claims 25-30, wherein said pectin has a molecular weight of between 70,000 and 90,000.

33. Use of a pectin according to any of claims 10-22 and 24 having a degree of blockiness of at least 10 %, a degree of esterification of at least 55 %, and a ΔCS of no more than 100 centipoise for increasing the storage stability of a comestible composition.

**Patentansprüche**

1. Verfahren zur Herstellung eines Pectins; das Verfahren umfasst den Schritt der Behandlung eines Ausgangspectins mit einem Blockhaftigkeitsgrad von mindestens 10 %, einem Veresterungsgrad von mindestens 55 % und einem ΔCS (Calciumempfindlichkeit) von mehr als 0 cP mit einer Pectinlyase unter Bedingungen, die zur Erzeugung eines verarbeiteten Pectins mit einem ΔCS, das niedriger ist als das ΔCS des Ausgangspectins, ausreichend sind.

2. Verfahren gemäss Anspruch 1, worin das Ausgangspectin einen Blockhaftigkeitsgrad von mindestens 20 % aufweist.

3. Verfahren gemäss Anspruch 1 oder 2, worin das Ausgangspectin einen Blockhaftigkeitsgrad von nicht mehr als 30 % aufweist.

4. Verfahren gemäss mindestens einem der Ansprüche 1 bis 3, worin das Ausgangspectin einen Veresterungsgrad von mindestens 55 % besitzt.

5. Verfahren gemäss Anspruch 4, worin das Ausgangspectin einen Veresterungsgrad zwischen 55 und 80 % besitzt.

6. Verfahren gemäss den Ansprüchen 1 bis 5, worin das Ausgangsmaterial ein ΔCS von mindestens 20 aufweist.

7. Verfahren gemäss mindestens einem der Ansprüche 1 bis 6, worin das verarbeitete Pectin ein ΔCS von nicht mehr als 20 cP aufweist.

8. Verfahren gemäss Anspruch 7, worin das verarbeitete Pectin ein ΔCS zwischen 0 und 20 cP aufweist.

9. Verfahren gemäss mindestens einem der Ansprüche 1 bis 8, worin das Ausgangspectin ein Molekulargewicht von nicht mehr als 240.000 besitzt.

**10.** Pectin, das nach einem Verfahren gemäss mindestens einem der Ansprüche 1 bis 9 erhältlich ist.

**11.** Pectin gemäss Anspruch 10, das einen Blockhaftigkeitsgrad von mindestens 10 %, einen Veresterungsgrad von mindestens 55 % und ein ∆CS von nicht mehr als 100 cP aufweist.

**12.** Pectin gemäss Anspruch 11, das ein ∆CS von nicht mehr als 30 cP aufweist.

**13.** Pectin gemäss Anspruch 12, das ein ∆CS zwischen 0 und 20 cP aufweist.

**14.** Pectin gemäss mindestens einem der Ansprüche 10 bis 13, das einen Blockhaftigkeitsgrad von mindestens 20 % besitzt.

**15.** Pectin gemäss Anspruch 14, das einen Blockhaftigkeitsgrad von nicht mehr als 30 % aufweist.

**16.** Pectin gemäss mindestens einem der Ansprüche 10 bis 15, das einen Veresterungsgrad von mindestens 60 % besitzt.

**17.** Pectin gemäss Anspruch 16, das einen Veresterungsgrad von bis zu 80 % besitzt.

**18.** Pectin gemäss Anspruch 17, das einen Veresterungsgrad zwischen 60 und 72 % besitzt.

**19.** Pectin gemäss mindestens einem der Ansprüche 10 bis 18, das ein Molekulargewicht von mindestens 40.000 besitzt.

**20.** Pectin gemäss Anspruch 19, das ein Molekulargewicht zwischen 50.000 und 200.000 besitzt.

**21.** Pectin gemäss mindestens einem der Ansprüche 10 bis 20, das einen Blockhaftigkeitsgrad zwischen 10 und 15, einen Veresterungsgrad zwischen 70 und 75 und ein ∆CS von nicht mehr als 18 aufweist.

**22.** Pectin gemäss Anspruch 21, das ein ∆CS von nicht mehr als 14 aufweist.

**23.** Verfahren zur Herstellung von Pectin, das die folgenden Schritte umfasst:

(a) Behandeln eines Pectins mit einem Blockhaftigkeitsgrad von mindestens 10 %, einem Veresterungsgrad von mindestens 70 % und einem ∆CS von mehr als 0 cP mit Pflanzenpectinmethylesterase unter Bedingungen, die zur Erzeugung eines entesterten Pectins mit einem Blockhaftigkeitsgrad von mindestens 10 %, einem Veresterungsgrad von mindestens 55 % und einem ∆CS von mehr als 0 ausreichend sind; und

(b) Behandeln des entesterten Produkts mit einer Pectinlyase unter Bedingungen, die zur Erzeugung eines Pectins mit einem Blockhaftigkeitsgrad von mindestens 10 % und einem ∆CS von nicht mehr als 20 cP ausreichend sind.

**24.** Pectin, das nach dem Verfahren gemäss Anspruch 23 erhältlich ist.

**25.** Nahrungsmittelzusammensetzung, die ein Pectin gemäss mindestens einem der Ansprüche 10 bis 22 und 24 mit einem Blockhaftigkeitsgrad von mindestens 10 %, einem Veresterungsgrad von mindestens 55 % und einem ∆CS von nicht mehr als 100 cP umfasst.

**26.** Nahrungsmittelzusammensetzung gemäss Anspruch 25, die ferner Casein umfasst.

**27.** Nahrungsmittelzusammensetzung gemäss Anspruch 26, worin die Nahrungsmittelzusammensetzung ein Getränk ist.

**28.** Nahrungsmittelzusammensetzung gemäss Anspruch 27, worin das Getränk ein angesäuertes Milchgetränk ist.

**29.** Nahrungsmittelzusammensetzung gemäss mindestens einem der Ansprüche 25 bis 28, worin das Pectin ein ∆CS von nicht mehr als 20 cP aufweist.

**30.** Nahrungsmittelzusammensetzung gemäss mindestens einem der Ansprüche 25 bis 29, worin das Pectin einen Blockhaftigkeitsgrad zwischen 15 und 25 % aufweist.

**31.** Nahrungsmittelzusammensetzung gemäss mindestens einem der Ansprüche 25 bis 30, worin das Pectin einen Veresterungsgrad zwischen 60 und 72 % aufweist.

**32.** Nahrungsmittelzusammensetzung gemäss mindestens einem der Ansprüche 25 bis 30, worin das Pectin ein Molekulargewicht zwischen 70.000 und 90.000 aufweist.

**33.** Verwendung eines Pectins gemäss mindestens einem der Ansprüche 10 bis 22 und 24 mit einem Blockhaftigkeitsgrad von mindestens 10 %, einem Veresterungsgrad von mindestens 55 % und einem $\Delta$CS von nicht mehr als 100 cP zur Erhöhung der Lagerungsstabilität einer Nahrungsmittelzusammensetzung.

**Revendications**

**1.** Procédé pour faire une pectine, ledit procédé comprenant l'étape de traitement d'une pectine de départ ayant un degré d'occurrence de blocs d'au moins 10 %, un degré d'estérification d'au moins 55 % et un $\Delta$CS (sensibilité au calcium) supérieur à 0 centipoise, avec une pectine lyase sous des conditions suffisantes pour produire une pectine traitée ayant un $\Delta$CS qui est inférieur au $\Delta$CS de ladite pectine de départ.

**2.** Procédé de la revendication 1, dans lequel ladite pectine de départ a un degré d'occurrence de blocs d'au moins 20 %.

**3.** Procédé de la revendication 1 ou 2, dans lequel ladite pectine de départ a un degré d'occurrence de blocs de pas plus que 30 %.

**4.** Procédé de l'une quelconque des revendications 1 - 3, dans lequel ladite pectine de départ a un degré d'estérification d'au moins 55 %.

**5.** Procédé de la revendication 4, dans lequel ladite pectine de départ a un degré d'estérification entre 55 % et 80 %.

**6.** Procédé des revendications 1 - 5, dans lequel ladite pectine de départ a un $\Delta$CS d'au moins 20.

**7.** Procédé de l'une quelconque des revendications 1 - 6, dans lequel ladite pectine traitée a un $\Delta$CS de pas plus que 20 centipoise.

**8.** Procédé de la revendication 7, dans lequel ladite pectine traitée a un $\Delta$CS entre 0 centipoise et 20 centipoise.

**9.** Procédé de l'une quelconque des revendications 1 - 8, dans lequel ladite pectine de départ a un poids moléculaire de pas plus que 240'000.

**10.** Pectine susceptible d'être obtenue par le procédé de l'une quelconque des revendications 1 - 9.

**11.** Pectine de la revendication 10, ayant un degré d'occurrence de blocs d'au moins 10 %, un degré d'estérification d'au moins 55 %, et un $\Delta$CS de pas plus que 100 centipoise.

**12.** Pectine de la revendication 11, ayant un $\Delta$CS de pas plus que 30 centipoise.

**13.** Pectine de la revendication 12, ayant un $\Delta$CS entre 0 centipoise et 20 centipoise.

**14.** Pectine de l'une quelconque des revendications 10 - 13, ayant un degré d'occurrence de blocs d'au moins 20 %.

**15.** Pectine de la revendication 14, ayant un degré d'occurrence de blocs de pas plus que 30 %.

**16.** Pectine de l'une quelconque des revendications 10 - 15, ayant un degré d'estérification d'au moins 60 %.

**17.** Pectine de la revendication 16, ayant un degré d'estérification entre 55 % et 80 %.

**18.** Pectine de la revendication 17, ayant un degré d'estérification entre 60 % et 72 %.

**19.** Pectine de l'une quelconque des revendications 10 - 18, ayant un poids moléculaire d'au moins 40'000.

**20.** Pectine de la revendication 19, ayant un poids moléculaire entre 50'000 et 200'000.

**21.** Pectine de l'une quelconque des revendications 10 - 20, ayant un degré d'occurrence de blocs entre 10 et 15, un degré d'estérification entre 70 et 75, et un $\Delta$CS de pas plus que 18.

**22.** Pectine de la revendication 21, ayant un $\Delta$CS de pas plus que 14.

**23.** Procédé pour faire une pectine, ledit procédé comprenant les étapes consistant à :

(a) traiter une pectine ayant un degré d'occurrence de blocs d'au moins 10 %, un degré d'estérification d'au moins 70 %, et un $\Delta$CS supérieur à 0 centipoise avec une pectine méthylestérase végétale sous des conditions suffisantes pour produire une pectine désestérifiée ayant un degré d'occurrence de blocs d'au moins 10 %, un degré d'estérification d'au moins 55 %, et un $\Delta$CS supérieur à 0 centipoise; et
(b) traiter ladite pectine désestérifiée avec une pectine lyase sous des conditions suffisantes pour produire une pectine ayant un degré d'occurrence de blocs d'au moins 10 % et un $\Delta$CS pas supérieur à 20 centipoise.

**24.** Pectine susceptible d'être obtenue par le procédé de la revendication 23.

**25.** Composition comestible comprenant une pectine selon l'une quelconque des revendications 10 - 22 et 24 ayant un degré d'occurrence de blocs d'au moins 10 %, un degré d'estérification d'au moins 55 %, et un $\Delta$CS de pas plus que 100 centipoise.

**26.** Composition comestible de la revendication 25, comprenant en outre de la caséine.

**27.** Composition comestible de la revendication 26, dans laquelle ladite composition comestible est une boisson.

**28.** Composition comestible de la revendication 27, dans laquelle ladite boisson est une boisson lactée acidifiée.

**29.** Composition comestible de l'une quelconque des revendications 25 - 28, dans laquelle ladite pectine a un $\Delta$CS de pas plus que 20 centipoise.

**30.** Composition comestible de l'une quelconque des revendications 25 - 29, dans laquelle ladite pectine a un degré d'occurrence de blocs entre 15 % et 25 %.

**31.** Composition comestible de l'une quelconque des revendications 25 - 30, dans laquelle ladite pectine a un degré d'estérification entre 60 % et 72 %.

**32.** Composition comestible de l'une quelconque des revendications 25 - 30, dans laquelle ladite pectine a un poids moléculaire entre 70'000 et 90'000.

**33.** Utilisation d'une pectine selon l'une quelconque des revendications 10 - 22 et 24 ayant un degré d'occurrence de blocs d'au moins 10 %, un degré d'estérification d'au moins 55 %, et un $\Delta$CS de pas plus que 100 centipoise pour augmenter la stabilité au stockage d'une composition comestible.